(19) **Europäisches Patentamt**
**European Patent Office**
**Office européen des brevets**

(11) Veröffentlichungsnummer: **0 150 835 B1**

(12) **EUROPÄISCHE PATENTSCHRIFT**

(45) Veröffentlichungstag der Patentschrift: **04.09.91**

(51) Int. Cl.⁵: **C07B 33/00**, C07C 63/26, C07C 69/82, C07C 51/265, C07C 67/313

(21) Anmeldenummer: **85100791.4**

(22) Anmeldetag: **26.01.85**

Die Akte enthält technische Angaben, die nach dem Eingang der Anmeldung eingereicht wurden und die nicht in dieser Patentschrift enthalten sind.

(54) **Verfahren zur Oxydation von flüssigen organischen Stoffen.**

(30) Priorität: **27.01.84 DE 3402807**

(43) Veröffentlichungstag der Anmeldung:
**07.08.85 Patentblatt 85/32**

(45) Bekanntmachung des Hinweises auf die Patenterteilung:
**04.09.91 Patentblatt 91/36**

(84) Benannte Vertragsstaaten:
**AT BE CH DE FR GB IT LI NL SE**

(56) Entgegenhaltungen:
EP-A- 0 087 670  DE-A- 1 442 821
GB-A- 2 014 473  US-A- 3 065 061
US-A- 3 534 090  US-A- 3 590 058

(73) Patentinhaber: **Bonsels, Paul, Dipl.-Ing.**
**Mariengrund 3**
**W-5216 Niederkassel(DE)**

Patentinhaber: **Neff, Ulrich**
**Gotenweg 60**
**W-5216 Niederkassel-Rheidt(DE)**

(72) Erfinder: **Bonsels, Paul, Dipl.-Ing.**
**Mariengrund 3**
**W-5216 Niederkassel(DE)**
Erfinder: **Neff, Ulrich**
**Gotenweg 60**
**W-5216 Niederkassel-Rheidt(DE)**

(74) Vertreter: **King, Hubert, Dipl.-Ing.**
**Kleiststrasse 8**
**W-5210 Troisdorf-Sieglar(DE)**

Rank Xerox (UK) Business Services

## Beschreibung

Die Erfindung bezieht sich auf ein Verfahren zur Oxydation von flüssigen organischen Stoffen, insbesondere von Gemischen aus Para-Xylol und Para-Toluylsäuremethylester mit sauerstoffhaltigen Gasen.

Bei den bisherigen Verfahren zur Oxydation von flüssigen organischen Stoffen ist es üblich, die Größe der Stoffaustauschfläche durch mechanische Maßnahmen wie etwa Rühren oder auch durch Verteilung des Gases mittels Düsen zu beeinflussen. Das Gas wird dabei in die Flüssigkeit eingeleitet und von dieser mehr oder weniger stark absorbiert.

Bei den dabei verwendeten Reaktoren handelt es sich um längliche vertikal oder auch horizontal angeordnete zylindrische Behälter mit längs oder quer angeströmten Kühlrohren zur Abfuhr der Reaktionswärme mittels eines geeigneten Kühlmediums (beispielsweise US-PS 30 65 061 und DE-OS 22 50 431). Durch die DE-PS 28 05 915 ist es auch bekannt, den Reaktor in mehrere aufeinanderfolgende Reaktionskammern zu unterteilen).

Durch die US-A-3 590 058 ist auch ein vertikal angeordneter Rohrreaktor mit einem haubenförmigen Kopfende bekannt, in welches eine Düse hineinragt. Dabei handelt es sich um einen Reaktor, bei dem die Oxydation, also der Reaktionsablauf, in einem Festbettkatalysator abläuft, da die mittels der Düse in den Kopfraum hinein zerstäubten Ausgangsmaterialien, d.h. die Flüssigphase und die Gasphase, im Kopfraum nicht miteinander reagieren, eine Reaktion vielmehr erst erfolgt, wenn das Zerstäubungsgemisch in den mit Festbettkatalysator gefüllten Reaktorbereich gelangt und durch diesen hindurchgeht.

Durch die EP-A-0 087 670 ist desweiteren ein vertikal angeordneter Reaktor bekannt, bei dem über eine Düse ein Flüssigkeitsstrahl nach abwärts in ein koaxial dazu angeordnetes zentrales Leitrohr führt und dadurch ein Umwälzvorgang in Gang gesetzt wird. Die Gasphase wird entweder nach Art der bekannten Blasensäulenreaktoren von unten nach oben in den Reaktor eingetragen oder aber im Bereich der Düse am oberen Ende des mit einem inerten Gas gefüllten Reaktorkopfendes.

Durch die DE-A-1 422 821 ist es schließlich bekannt, die kontinuierliche Reaktion von strömungsfähigen Medien in einem Reaktionsgefäß durchzuführen, indem ein Gemisch der miteinander reagierenden Medien mittels einer in den Strömungskreis einmündenden Düse im Reaktorgefäß unter Druck und mit hoher Geschwindigkeit umgewälzt und dabei eine Rückführung des Reaktionsgemisches durch das Reaktionsgebiet erzielt wird, und wobei die Rückführung durch das Reaktionsgebiet mittels Veränderung der Querschnittsfläche der Düse beeinflusst wird.

Infolge der undefinierten Strömungsverhältnisse sowohl der Gasphase als auch der Flüssigkeit ist bei diesen Einrichtungen weder eine definierte Verweil- und Reaktionszeit noch eine exakte Temperaturführung zu erreichen, so daß es - insbesondere auch wegen des schlechten Wärmeübergangs bei längs wie quer angeordneten Kühlrohren - zu örtlichen, die thermische Belastbarkeit der Flüssigkeit übersteigenden, Erhitzungen kommt. Die verhältnismäßig geringe vorhandene Stoffaustauschfläche hat darüber hinaus den Nachteil eines hohen erforderlichen Aufwandes an Reaktionsgas und damit an Energie und führt in Verbindung mit dem undefinierten und praktisch nicht beeinflußbaren Verhalten des Reaktionsgases beim Durchgang durch die Flüssigkeitssäule zu verhältnismäßig großvolumigen, ein großes Totvolumen aufweisenden und großen Platzbedarf sowie hohe Investitionskosten erfordernden Reaktoren.

Die Erfindung macht es sich zur Aufgabe, bei einem Verfahren zur Oxydation von flüssigen organischen Stoffen, insbesondere von Gemischen aus Para-Xylol und Para-Toluylsäuremethylester mit sauerstoffhaltigen Gasen unter Berücksichtigung der zahlreichen bedeutsamen Faktoren wie beispielsweise thermische Belastbarkeit der Flüssigkeit, Dauer der Verweilzeit, Größe des Absorbtionskoeffizienten, Art der chemischen Reaktion, Druck und Temperatur die Nachteile der bekannten Verfahren zu vermeiden und dadurch bei geringeren Investitionskosten und geringerem Platzbedarf eine höhere Ausbeute zu erzielen.

Diese Aufgabe wird erfindungsgemäß dadurch gelöst, daß die Oxydation in wenigstens einem vertikal angeordneten Rohrreaktor durchgeführt wird, der an seinem oberen Ende einen von Einbauten freien Impulsraum aufweist, in den eine auf dem Reaktor angeordnete und an die Flüssigphase und an die Gasphase angeschlossene Rohrreaktorstrahldüse mündet, wobei die Rohrreaktorstrahldüse fortlaufend mit der von einer Pumpe geförderten und in einem Wärmetauscher auf Reaktionstemperatur gebrachten Flüssigphase über Leitungen als zentralem Treibstrahl und über weitere Leitungen als ringförmigem Nebenstrahl sowie fortlaufend mit der über eine Pumpe geförderten und in einem Wärmetauscher auf die erforderliche Temperatur gebrachten Gasphase über eine Leitung in den zwischen dem Treibstrahl und dem Nebenstrahl gebildeten Ringraum beschickt wird, daß die Flüssigphase und die Gasphase nach Durchströmen der Rohrreaktorstrahldüse im anschließenden Impulsraum fortlaufend innig miteinander vermischt werden und das im Impulsraum gebildete Reaktionsprodukt fortlaufend durch die an den Impulsraum anschließende Kühlzone des Reaktors hindurchgeführt wird, und daß schließlich das Oxydationsprodukt hinter der Kühlzone des Reaktors aus der an diese anschließenden Produktabführungszone und gleichzeitig das im wesentlichen aus inerten Bestandteilen bestehende Restgas aus dem Impulsraum abgezogen werden, wobei die Zufuhr der Flüssigphase und der Gasphase sowie der Abzug des Oxydationsproduktes und des Restgases so gesteuert werden, daß sich der Flüssigkeitsspiegel im Reaktor möglichst genau in Höhe des oberen Endes der Rohre bzw. des oberen Bodens befindet.

Durch die erfindungsgemäße Maßnahme wird nicht nur eine exakte Produktführung bezüglich Flüssigkeitsmenge und Flüssigkeitszusammensetzung sowie eine exakte Gasdosierung bezüglich Menge und Zusammensetzung ermöglicht, sondern durch eine wesentlich vergößerte Stoffaustauschfläche zwischen Flüssigkeit, Gas und Katalysator auch eine wesentlich verbesserte Reaktionsführung sowie eine erhebliche Verringerung der benötigten Menge des Katalysators erzielt. Auch ist eine genaue Temperaturführung durch direkte und wirksame Abführung der Reaktionsenthalpie sowie eine der ungewollten Bildung von Nebenprodukten entgegenwirkende genaue Steuerung der Verweilzeit möglich.

In zwckmäßiger weiterer Ausgestaltung der Erfindung ist vorgesehen, den Druck im System mittels einer in einer an den Impulsraum angeschlossenen Abgasleitung angeordneten Regeleinrichtung zu steuern. Von Vorteil ist es dabei, einen Teil des Oxydationsproduktes und/oder der Gasphase, für die insbesondere reiner Sauerstoff verwendet werden kann, während einer beliebig veränderbaren Verweildauer im Oxydationskreislauf zu halten, wodurch es ebenfalls möglich ist, den Reaktionsablauf in der gewünschten Weise zu beeinflussen und der Bildung der unerwünschten Nebenprodukte gezielt entgegenzuwirken, wie bei der Durchführung der Oxydation in zwei oder mehreren beliebig miteinander kombinierbaren hintereinandergeschalteten Oxydationsstufen, durch die sich der Prozeß bezüglich Konzentration der Reaktionspartner, Mengenverhältnisse, Temperaturführung und Abführen der Reaktionswärme optimal gestalten lässt. Der Verbrauch an Reaktionsgas kann dabei günstig beeinflußt werden, indem das Abgas der nachfolgenden Reaktionsstufen der jeweils vorhergehenden Stufe wieder zugeführt und der Sauerstoffgehalt dieser vorhergehenden Stufe durch Zufuhr der noch erforderlichen Menge Frischgas eingestellt wird. Für das schließlich aus dem Prozeß ausgeschleuste Abgas ist vorgesehen, dieses einer Reinigungsanlage zuzuführen. Da die Bildung explosiver Gemische während des Reaktionsablaufs nicht völlig ausgeschlossen werden kann, ist erfindungsgemäß vorgesehen, den Impulsraum mittels radioaktiver Strahlen zu ionisieren.

Die Erfindung ist in der Zeichnung in Ausführungsbeispielen gezeigt und wird anhand dieser nachfolgend beschrieben.
Es zeigen

Figur 1   in schematischer Darstellung einen Rohrreaktor mit Rohrreaktorstrahldüse

Figur 2   mehrere Rohrreaktoren mit Rohrreaktorstrahldüse in Hintereinanderschaltung.

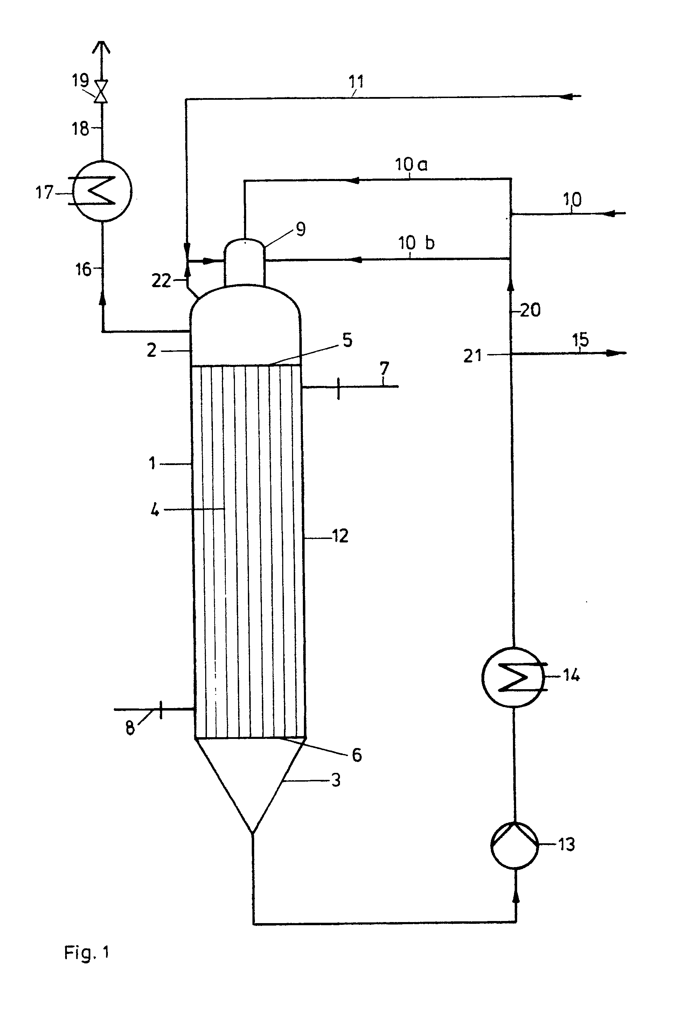

In Figur 1 weist der Rohrreaktor 1 an seinem oberen Ende den Impulsraum 2 und an seinem unteren Ende die Produktabführungszone 3 auf. Zwischen diesen befinden sich die Rohre 4, deren Zwischenräume zum Impuisraum 2 und zur Produktabführungszone 3 hin mittels der Böden 5 und 6 als Kühlzone 12 verschlossen sind und zwecks Zu- und Abfuhr von Kühlmedium an die Leitungen 7 und 8 angeschlossen sind.

Auf dem Rohrreaktor 1 ist in den Impulsraum 2 mündend die Rohrreaktorstrahldüse 9 angeordnet. Diese wird über die Leitungen 10 und 10 a mit der von einer nicht gezeigten Pumpe geförderten und in einem ebenfalls nicht gezeigten Wärmeaustauscher auf die erforderliche Temperatur eingestellten Flüssigphase als zentralem Treibstrahl und über die Leitungen, 10 und 10 b als ringförmigem Nebenstrahl sowie über die Leitung 11 in einen zwischen dem Treibstrahl und dem Nebenstrahl gebildeten nicht gezeigten Ringraum mit der beispielsweise ebenfalls über eine Pumpe geförderten und in einem nicht gezeigten Wärmeaustauscher optimal temperierten Gasphase beschickt. Nach Durchströmen der Rohrreaktorstrahldüse 9 werden Flüssig- und Gasphase im Impulsraum 2 innig vermischt und gelangen unmittelbar in die Kühlzone 12. Das Oxydationsprodukt wird von der Pumpe 13 über den Wärmetauscher 14 und die Rohrleitung 15 weitergeleitet.

Das im wesentlichen aus inerten Bestandteilen bestehende Restgas wird über die Leitung 16 zum Brüden-Kondensator 17 geleitet, wo das während der Reaktion sich gebildete Reaktionswasser abgeschieden wird, und gelangt nach Entspannung in eine nicht gezeigte Abluftreinigung. Zur Steuerung des Druckes im System ist in der Abluftleitung 18 die Regelung 19 vorgesehen.

Der Oxydationsvorgang kann in weiten Bereichen gesteuert werden. Dazu werden in den Rohrleitungen entsprechende Steuer- und Regelventile vorgesehen. Durch die Verbindung der Produktabfuhrleitüng 15 mit der Leitung 10 mittels der Leitung 20 und Anordnung eines Steuer- bzw. Regelventils an der Verbindungsstelle 21 kann bezweckt und erreicht werden, daß ein mehr oder weniger großer Teil des Oxydationsproduktes für einen beliebig festlegbaren Zeitraum im Oxydationskreislauf gehalten wird. Ebenso kann durch die Verbindungsleitung 22 zwischen Impulsraum 2 und Leitung, 11 mit nicht gezeigtem Steuer- bzw. Regelventil bezweckt und erreicht werden, daß ein mehr oder weniger großer Teil der Gasphase mehr oder weniger lange im Oxydationskreislauf gehalten wird.

Da im Rohrreaktor 1 keinerlei Toträume vorhanden sind und eine einstellbare hohe Strömungsgeschwindigkeit herrscht, wird die Reaktionswärme sowohl in der Kühlzone 12 als auch durch den außenliegenden Wärmetauscher 14 schnell und wirksam abgeführt. Im Sinne einer optimalen Kühlwirkung, wird die Steuerung bzw. Regelung des Prozeßablaufs so getroffen, daß sich der Flüssigkeitsspiegel im Rohrreaktor möglichst genau in Höhe des oberen Endes der Rohre 4 bzw. des oberen Bodens 5 befindet.

Das im Vorstehenden beschriebene Verfahren zur Oxydation in nur einem Rohrreaktor läßt sich durch die Kombination mehrerer solcher Rohrreaktoren wesentlich verbessern. So läßt sich durch mehrere Reaktionsstufen der Prozeß bezüglich Konzentration der Reaktionspartner, Mengenverhältnisse, Temperaturverhältnisse sowie Abführen der Reaktionswärme optimal gestalten, wodurch die Reaktion in allen wesentlichen Parametern beherrschbar wird und damit Ausbeute und Selektivität gegenüber den bekannten Verfahren wesentlich verbessert wird.

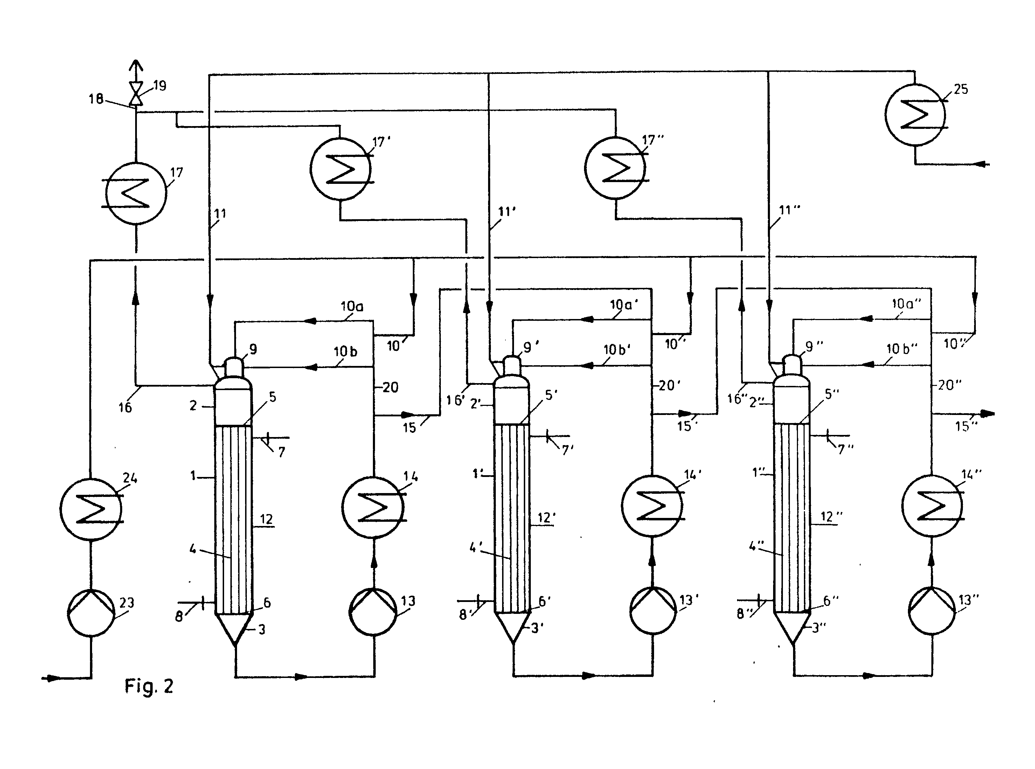

Bei der in Figur 2 gezeigten Anwendung mehrerer Oxydatoren im mehrstufigen Prozeß sind gleiche Teile wieder mit den gleichen Bezugszeichen versehen.

Die von der Pumpe 23 über den Wärmetauscher 24 durch die Leitungen 10, 10', 10" und die von den Pumpen 13, 13', 13" über die Leitung 20, 20', 20" geförderte Kreislaufmenge an Oxydationsprodukt können durch nicht gezeigte Steuer- und Regelorgane in beliebigen Verhältnissen variiert werden, so daß in jeder Stufe sowohl reinem Ausgangsprodukt wie auch jede beliebige Mischung von Ausgangsprodukt und Oxydationsprodukt oxydiert werden kann. Vorteilhaft kann die Anordnung und Ausbildung so getroffen werden, daß die verschiedenen Stufen in beliebiger Kombination miteinander betrieben werden können, d.h. daß einzelne Stufen auch ausgeschaltet werden können.

Die Möglichkeit der beliebigen Variation ist auch bezüglich der Menge und Zusammensetzung des Reaktionsgases gegeben, dessen Temperierung im Wärmeaustauscher 25 erfolgt. Beispielsweise kann der Reaktor 1 mit Ausgangsprodukt beschickt und über die Reaktoren 1', 1" usw. zum Zielprodukt oxydiert werden. Das Oxydationsgas kann dabei im Gegenstrom geführt werden, d.h. die größte $O_2$-Konzentration wird in der letzten Stufe angeboten und zur ersten Stufe hin abgebaut oder aber je nach $O_2$-Gehalt nach jeder Stufe direkt in die Reinigungsanlage geleitet. Durch separate Frischgasbeimengung kann jede Stufe in ihrem Oxydationsablauf eingestellt werden, wobei die einzelnen Stufen durch Mischen von Frischprodukt und Umlaufprodukt auf günstigste Konzentration regulierbar sind.

Bei dem für die Oxydation verwendeten sauerstoffhaltigen Gas kann es sich sowohl um ein mehr oder weniger große inerte Anteile aufweisendes Gas als auch um reinen Sauerstoff handeln, wobei die Steuerung bzw. Regelung des Ablaufs des Oxydations-Prozesses ohne Schwierigkeit in der jeweils erforderlichen Weise angepaßt werden kann. Bei entsprechender Prozeßführung hat die Verwendung reinen Sauerstoffes als Reaktionsgas den Vorteil, daß sich die Abgasmenge zumindest wesentlich verringern, u.U. sogar ganz vermeiden läßt.

Die Reaktionswärme wird unmittelbar am Ort ihrer Entstehung, nämlich in den Kühlzonen 12, 12', 12" sowie in den außenliegenden Wärmeaustauschern 14, 14', 14" usw. abgeführt. Kühlzonen und Wärmeaustauscher werden selbstverständlich einzeln regulierbar ausgebildet. Da sie vom Produkt gleichsam nach Art einer Kolbenströmung durchflossen werden, werden örtliche Überhitzungen vermieden.

Die Abluft des letzten Reaktors kann, nachdem das Reaktionswasser auskondensiert ist, der vorhergehenden Stufe als Reaktionsgas dienen. Die $O_2$-Konzentration kann dazu erforderlichenfalls mit Frischgas angehoben werden, das Abgas dieser vorhergehenden Stufe kann in gleicher Weise wiederum nach Auskondensieren des Reaktionswassers der ihr vorhergehenden Stufe zugeführt werden usw., wobei in jeder vorhergehenden Stufe der $O_2$-Gehalt mit Frischgas eingestellt werden kann, bis schließlich das Abgas der ersten Stufe nach Auskondensieren des Reaktionswassers in die Reinigungsanlage geführt wird.

Wie bei den bisher gebräuchlichen Oxydatoren kann auch bei dem hier beschriebenen Verfahren die Bildung explosiver Gemische in der Gasphase nicht unbedingt ausgeschlossen werden. Das Volumen des Gasraumes beträgt hier jedoch nur einen Bruchteil des Gasvolumens der bisher eingesetzten Apparaturen, wodurch die Gefahr schon erheblich gemindert ist. Um einen vollständigen Explosionsschutz zu gewährleisten, ist erfindungsgemäß vorgesehen, im Impulsraum eines jeden Rohrreaktors einen Ionisator in Form einer radioaktiven Quelle, insbesondere eines α-Strahlers anzuordnen.

## Patentansprüche

1. Verfahren zur Oxydation von flüssigen organischen Stoffen, insbesondere von Gemischen aus Para-Xylol und Para-Toluylsäuremethylester, mit sauerstoffhaltigen Gasen, dadurch gekennzeichnet, daß man die Oxydation in wenigstens einem vertikal angeordneten Rohrreaktor (1) durchführt, der an seinem oberen Ende einen von Einbauten freien Impulsraum (2) aufweist, in den eine auf dem Reaktor angeordnete und an die Flüssigphase und an die Gasphase angeschlossene Rohrreaktorstrahldüse (9) mündet, wobei die Rohrreaktorstrahldüse (9) fortlaufend mit der von einer Pumpe geförderten und in einem Wärmetauscher auf Reaktionstemperatur gebrachten Flüssigphase über die Leitungen (10/10a) als zentralem Treibstrahl und über die Leitungen (10), (10b) als ringförmigem Nebenstrahl sowie fortlaufend mit der über eine Pumpe geförderten und in einem Wärmetauscher auf die erforderliche Temperatur gebrachten Gasphase über die Leitung (11) in den zwischen dem Treibstrahl und dem Nebenstrahl gebildeten Ringraum beschickt wird, die Flüssigphase und die Gasphase nach Durchströmen der Rohrreaktorstrahlduse (9) im anschließenden Impulsraum (2) fortlaufend innig miteinander vermischt werden und das im Impulsraum (2) gebildete Reaktionsprodukt fortlaufend durch die an den Impulsraum anschließende Kühlzone (12) des Reaktors (1) hindurchgeführt wird, und schließlich das Oxydationsprodukt hinter der Kühlzone des Reaktors aus der an diese anschließenden Produktabführungszone (3) und gleichzeitig das im wesentlichen aus inerten Bestandteilen bestehende Restgas aus dem Impulsraum (2) abgezogen werden, wobei die Zufuhr der Flüssigphase und der Gasphase sowie der Abzug des Oxydationsprodukts und des Restgases so gesteuert werden, daß sich der Flüssigkeitsspiegel im Reaktor möglichst genau in Höhe des oberen Endes Rohre (4) bzw. des oberen Bodens (5) befindet.

2. Verfahren nach Anspruch 1, dadurch gekennzeichnet, daß der Druck im System mittels einer Regeleinrichtung (19) gesteuert wird, die in einer an den Impulsraum angeschlossenen Abgasleitung (18) angeordnet ist.

3. Verfahren nach Anspruch 1 oder 2, dadurch gekennzeichnet, daß ein Teil des Oxydationsprodukts während einer beliebig veränderbaren Verweildauer im Oxydationskreislauf gehalten wird.

4. Verfahren nach einem der Ansprüche 1 bis 3, dadurch gekennzeichnet, daß ein Teil der Gasphase während einer beliebig veränderbaren Verweildauer im Oxydationskreislauf gehalten wird.

5. Verfahren nach einem der Ansprüche 1 bis 4, dadurch gekennzeichnet, daß als Gasphase reiner Sauerstoff verwendet wird.

6. Verfahren nach einem der Ansprüche 1 bis 5, dadurch gekennzeichnet, daß die Oxydation in zwei oder in mehreren beliebig miteinander kombinierbaren hintereinandergeschalteten Stufen durchgeführt wird.

7. Verfahren nach Anspruch 6, dadurch gekennzeichnet, daß das Abgas der zweiten und eventueller weiterer Stufen nach Auskondensieren des Reaktionswassers der jeweils vorhergehenden Stufe zugeführt und der Sauerstoffgehalt dieser vorhergehenden Stufe bzw. Stufen jeweils durch Zufuhr von Frischgas neu eingestellt wird.

8. Verfahren nach Anspruch 7, dadurch gekennzeichnet, daß das Abgas, das aus dem Prozeß ausgeschleust wird, nach Auskondensieren des Reaktionswassers einer Reinigungsanlage zugeführt wird.

9. Verfahren nach einem der Ansprüche 1 bis 8, dadurch gekennzeichnet, daß der Impulsraum mittels radioaktiver Strahlen, vorzugsweise α-Strahlen, ionisiert wird.

**Claims**

1. A process for the oxidation of liquid organic substances, in particular of compounds of para-xylol and para-toluylic acid methylester, with oxygenous gases, characterized in that oxidation is performed in at least one vertically positioned tubular reactor (1) which has at its top end an impulse space (2) free from built-in components, into which a tubular reactor jet nozzle (9) located on the reactor and connected to the liquid phase and to the gas phase leads, whereby the tubular reactor jet nozzle (9) of the tubular reactor is charged continuously with the liquid phase which is fed by a pump and brought to reaction temperature in a heat exchanger via the lines (10), (10a) as the central jet and via the lines (10), (10b) as an annular secondary jet as well as continuously with the gas phase which is fed by a pump and brought to the necessary temperature in a heat exchanger via the line (11) into the annular space formed by the central jet and the secondary jet, the liquid phase and the gas phase are continuously mixed intimately with each other after flowing through the tubular reactor jet nozzle (9) in the adjacent impulse space (2) and the reaction product formed in the impulse space (2) is fed continuously through the cooling zone (12) of the reactor (1) adjacent to the impulse space, and the oxidation product behind the cooling zone of the reactor is finally drawn from the product discharge zone (3) adjacent to this and at the same time the residual gas which consists essentially of inert components is withdrawn from the impulse space (2), with the feed of the liquid phase and of the gas phase and the withdrawal of the oxidation product and of the residual gas being controlled by keeping the liquid level in the reactor as exactly as possible at the level of the top end of the tubes (4) or of the top plate (5).

2. Process according to claim 1, characterized in that the pressure in the system is controlled by an automatic controller (19) located in a waste gas line (18) connected to the impulse space.

3. Process according to claim 1 or claim 2, characterized in that part of the oxidation product is retained in the oxidation cycle for a period that can be altered at random.

4. Process according to one of claims 1 through 3, characterized in that part of the gas phase is retained in the oxidation cycle for a period that can be altered at random.

5. Process according to one of claims 1 through 4, characterized in that pure oxygen is used as the gas phase.

6. Process according to one of claims 1 through 5, characterized in that oxidation is performed in two or more stages connected in series that can be combined with one another at random.

7. Process according to claim 6, characterized in that the waste gas from the second and any further stages is fed to the previous respective stage after complete condensation of the reaction water and the respective oxygen content of this preceding stage or of these preceding stages is re-set by adding fresh gas.

8. Process according to claim 7, characterized in that the waste gas resulting from the outward transfer of the process is fed to a scrubbing system after complete condensation of the reaction water.

9. Process according to one of claims 1 through 8, characterized in that the impulse space is ionized by means of radioactive beams, preferably alpha beams.

**Revendications**

1. Procédé d'oxydation de matières organiques liquides, en particulier de mélanges à base de para-xylol et d'ester méthylique d'acide para-toluique, avec des gaz contenant de l'oxygène, procédé caractérisé par le fait que l'on réalise l'oxydation dans un réacteur tubulaire au moins disposé verticalement (1), réacteur qui présente à son extrémité supérieure une chambre à impulsions exempte de chicanes (2) dans laquelle débouche un éjecteur de réacteur tubulaire (9) disposé sur le réacteur et raccordé à la phase liquide et à la phase gazeuse; l'éjecteur du réacteur tubulaire (9) est chargé en continu de la phase liquide refoulée via une pompe et amenée à la température de réaction dans un échangeur de chaleur par l'intermédiaire des canalisations (10), (10a) comme jet de propulsion central et par l'intermédiaire des canalisations (10), (10b) comme jet secondaire annulaire; l'éjecteur est également chargé en continu de la phase gazeuse refoulée via une pompe et amenée à la température nécessaire dans un échangeur de chaleur par l'intermédiaire de la canalisation (11) dans l'espace annulaire formé entre le jet de propulsion et le jet secondaire; la phase liquide et la phase gazeuse sont mélangées en continu intimement l'une à l'autre grâce au passage de l'éjecteur du réacteur tubulaire (9) dans la chambre à impulsions attenante (2), et le produit de la réaction formé dans la chambre à impulsions (2) est amené en continu à travers la zone de refroidissement (12) du réacteur (1) attenante à la chambre à impulsions, et le produit d'oxydation derrière la zone de refroidissement du réacteur est finalement soutiré de la zone d'évacuation des produits attenante (3) et le gaz résiduel constitué essentiellement de composants inertes simultanément soutiré de la chambre à impulsions (2), l'apport de la phase liquide et de la phase gazeuse ainsi que le retrait du produit d'oxydation et du gaz résiduel étant commandés de sorte que le niveau de liquide dans le réacteur se situe le plus précisément possible au niveau de l'extrémité supérieure du tuyau (4) et/ou du fond supérieur (5).

2. Procédé selon la revendication 1, caractérisé par le fait que la pression dans le système est commandée au moyen d'un dispositif de régulation (19) qui est disposé dans une canalisation de gaz d'échappement (18) raccordée à la chambre à impulsions.

3. Procédé selon les revendications 1 ou 2, caractérisé par le fait qu'une partie du produit d'oxydation est maintenu dans le cycle d'oxydation pendant une durée de séjour modifiable au choix.

4. Procédé selon l'une des revendications de 1 à 3, caractérisé par le fait qu'une partie de la phase gazeuse est maintenue dans le cycle d'oxydation pendant une durée de séjour modifiable au choix.

5. Procédé selon l'une des revendications de 1 à 4, caractérisé par le fait que l'on utilise comme phase gazeuse de l'oxygène pur.

6. Procédé selon l'une des revendications de 1 à 5, caractérisé par le fait que l'oxydation est réalisée en deux étapes ou plus, disposées l'une derrière l'autre et combinables au choix.

7. Procédé selon la revendication 6, caractérisé par le fait que les gaz d'échappement de la deuxième étape, et éventuellement d'autres étapes, sont amenés à l'étape respectivement précédente après l'évaporation par condensation de l'eau de réaction, et que la teneur en oxygène de cette étape précédente ou d'étapes précédentes est réglé une nouvelle fois par l'apport de gaz frais.

8. Procédé selon la revendication 7, caractérisé par le fait que les gaz d'échappement éclusés au dehors du processus sont amenés à une installation d'épuration après évaporation par condensation de l'eau de réaction.

9. Procédé selon l'une des revendications de 1 à 8, caractérisé par le fait que la chambre à impulsions est ionisée au moyen de rayons radioactifs, de préférence des rayons α.

Fig. 1

Fig. 2